# EUROPEAN PATENT APPLICATION

(11) **EP 4 431 070 A1**
(43) Date of publication of application: **18.09.2024**
(21) Application number: 22889499.4
(22) Date of filing: 21.10.2022
(51) Int. Cl.: A61F 13/47, A61F 13/49

(54) **ABSORBENT ELEMENT HAVING A MOISTURE BARRIER**

(30) Priority: 08.11.2021 ES 202100439 U
(71) Applicant: Fundación Profesor Novoa Santos, 15006 A Coruña (ES)
(72) Inventor: FERNÁNDEZ VARELA, José Luis, 15159 Montrove, Oleiros La Coruña (ES)
(74) Representative: ABG Intellectual Property Law, S.L.
(86) International application number: PCT/ES2022/000045
(87) International publication number: WO 2023/079193

(57) **Abstract**

The present invention relates to an absorbent element having a moisture barrier, said element consisting of a sanitary garment in the form of a special diaper for use by people suffering from urinary incontinence, characterised in that it is formed by a normal diaper and a substantially rectangular additional piece that is firmly attached to the normal diaper, wherein one end of the additional piece is positioned close to the genital area that becomes wet, the upper end thereof is positioned close to the end of the normal diaper, and the sides thereof are aligned with the sealing bands of the normal diaper.

## Description

### OBJECT OF THE INVENTION

The present invention describes a sanitary garment for use by people suffering from urinary incontinence with normal, reduced or dependent mobility and users of anatomical absorbent elements or pull-up diaper-type absorbent elements.

### FIELD OF THE ART OF THE INVENTION

The invention herein proposed belongs to the sector relating to everyday necessities of life, in the chapter on health and protection in reference to medical sciences and hygiene, of the International Patent Classification (IPC), involved directly in the clinical and pharmaceutical industry concerning the manufacture of special garments and accessories for people with urinary incontinence (children, adults, the elderly).

### BACKGROUND OF THE INVENTION

The technologies of companies dedicated to the manufacture of absorbent elements are subject to multiple improvements and advances for the benefit of users, with the ultimate goal thereof being to offer superior absorption that keeps the skin in contact with the absorbent element dry. The main purpose of diapers is to absorb urine released by the incontinent person to prevent contact with moisture. One of the main drawbacks of diapers is the return moisture that arises when pressing on the diaper when it is soaked with urine.

The absorbent elements on the market have an absorption of between 860 ml and 1600 ml; this amount of urine makes up 80% of the weight of the diaper when it is changed.

In one study, the return moisture in different brands of diapers is between 0.7% and 4.9%. An absorbent element of 1200 ml would have a return moisture of between 12 ml and 60 ml. This means that the skin of the user of the absorbent element, in the pressure areas, will be in contact with urine to a greater or lesser extent.

This entails discomfort for the user caused by moisture and in many cases skin irritation, such as maceration, dermatitis, and even bedsores, occurs due to the pH of the urine.

In the current state of the art, some registered prior art documents that are designed to prevent moisture from contacting the skin are known, particularly for diaper users such as small children, adults with some sort of disability, and the elderly.

Basically, they describe special diapers with a high absorption capacity as they have several layers of absorbent material. Electronic devices which, as a complement to normal diapers, detect when the diaper starts to get wet and send notifications to nurses or caregivers, which are centralised in a wireless control centre, are also known. In other words, this device prevents the patient from spending a long time in a wet diaper.

In view of the known prior art documents, the inventor describes a solution that represents an interesting novelty shown as a special diaper provided with a moisture barrier to prevent damage to the skin.

### BRIEF DESCRIPTION OF THE INVENTION

The inventor proposes the incorporation, in a regular diaper formed by several layers of absorbent material, of a layer of the same material made up of a waterproof area in direct contact with the main diaper and another thin cellulose area that will be in contact with the skin. This layer overlaps the main absorbent element, extends from the front area of the perineum, where it is sealed, to the end of the diaper, and reaches the sealing area of the main diaper on the sides.

Therefore, only the front area of the absorbent element where the genital area is located would come into contact with urine. This area only represents 25% of the absorbent element, with the remaining 75% being protected from urine and return moisture by the barrier proposed in this invention.

The urine absorbed by the main diaper is kept under the overlapping layer, with the cellulose of this barrier, which is in contact with the skin, thereby always being clean and dry with a greater feeling of comfort and preventing problems derived from moisture, such as maceration, dermatitis, and even bedsores.

### BRIEF DESCRIPTION OF THE DRAWINGS

Three figures, which are considered sufficient for the proper interpretation of the invention, are included.

### Figure 1

This figure schematically depicts one of the main components of the diaper of the invention.
1. Normal diaper
2. Absorbent material
5. Waterproof material
4. Area to be protected
5. Genital area

### Figure 2

This figure schematically depicts the other component of the diaper of the invention.
6. Additional piece
7. Thin cellulose sheet

### Figure 3

This figure depicts the absorbent element of the invention once the additional piece has been incorporated in the normal diaper.
5.1. Genital area that becomes wet
8. Absorbent element with a barrier
9. Sealing band

### DETAILED EXPLANATION OF AN EMBODIMENT OF THE INVENTION

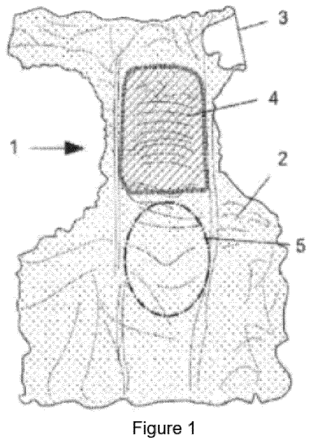

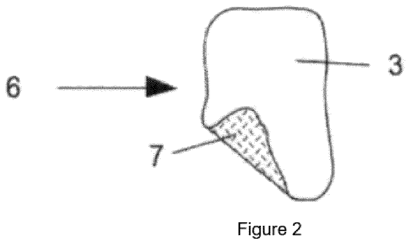

The present invention relates to an absorbent element having a moisture barrier (8) (Figure 2), said absorbent element consisting of a sanitary garment in the form of a special diaper for use by people suffering from urinary incontinence which, in an embodiment preferred by its inventor, is shown as a normal diaper (1) (Figure 1) of the types made up of a plurality of layers of absorbent material which are arranged on the side of the user's skin, with there being, at the outer end, a layer of waterproof material (3) to prevent wetting the bedding or clothing in general. The perimeter edge is suitably sealed, the garment having quick fastening means based on Velcro^{®} straps or the like.

There is indicated in this normal diaper (1) an area to be protected (4), which has been marked with a discontinuous line, intended for receiving the additional piece (6), depicted in (Figure 2), which has a substantially rectangular shape and is made up of a layer of waterproof material (3) and a thin cellulose sheet (7). The genital area (5), which is where the urine of the user is typically found, and therefore the wettest area, is also marked with a discontinuous line.

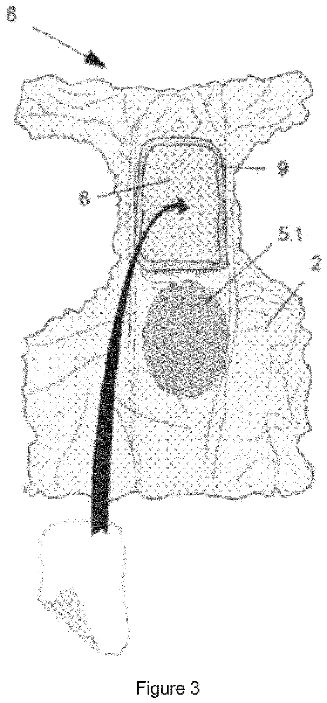

The diaper of the invention (8) in which the additional piece (6) has already been incorporated in the normal diaper (1) is depicted in (Figure 3). The same figure suggests the movement of said piece to the intended location, with the particularity that the position of the layers thereof has been inverted, with the waterproof material (3) thereof being in contact with the absorbent material (2) of the normal diaper and the thin cellulose sheet (7) being visible.

Furthermore, it can be seen that the perimeter edge of the additional piece has been sealed by the band (9) with the lower end thereof being positioned close to the genital area that becomes wet (5.1), the upper end thereof being positioned close to the end of the diaper, and the sides thereof being aligned with the sealing bands of the normal diaper.

In these conditions, as indicated in the preceding paragraphs, the skin of the user would only come into contact with the urine in the front area of the absorbent element where the genital area is located. This area represents about 25% of the absorbent element, with the remaining 75% being protected from urine and return moisture by the barrier proposed in this invention, which is highly beneficial in the sense of preventing macerations, dermatitis, and even bedsores.

It is understood that the absorbent element with a barrier (8) of the invention, intended to be manufactured in different sizes, is very suitable for use in any private home where there are small children or elderly people, and obviously in all types of hospitals, retirement homes, daycare centres, and similar establishments.

It is not considered necessary to further describe the content of this description for a person skilled in the art to be able to understand the scope and advantages derived from the invention, as well as to develop and put into practice the object thereof. However, it should be understood that the invention has been described according to preferred embodiments thereof, and modifications can be contemplated without this resulting in or entailing any alteration whatsoever of the basis of said invention. In other words, the terms in which this description of the invention has been set forth should always be interpreted in a broad and non-limiting manner.

## Claims

1. An absorbent element having a moisture barrier (8), said absorbent element consisting of a sanitary garment in the form of a special diaper for use by people suffering from urinary incontinence, **characterised in that** it is formed by a normal diaper (1) and a substantially rectangular additional piece (6) that is fixed to the normal diaper, wherein one end of the additional piece is positioned close to the genital area that becomes wet (5.1), the upper end thereof closer to the end of the normal diaper, and the sides thereof are aligned with the sealing bands of the normal diaper, with the normal diaper being of the types made up a plurality of layers of absorbent material (2) and a layer of waterproof material (3) and the additional piece (6) having a layer of waterproof material (3) and a thin cellulose sheet (7).

2. The absorbent element having a moisture barrier according to claim 1, **characterised in that** the additional piece (6) has a perimeter sealing band (9).

3. The absorbent element having a moisture barrier according to claim 1, **characterised in that** the additional piece (6) is fixed to the normal diaper (1), placing the layer of waterproof material (3) thereof in contact with the absorbent material (2) of the normal diaper.

4. The absorbent element having a moisture barrier according to claim 1, **characterised in that** it is manufactured in different sizes.
